# EUROPÄISCHE PATENTSCHRIFT

(11) **EP 0 558 511 B1**
(45) Veröffentlichungstag und Bekanntmachung des Hinweises auf die Patenterteilung: **17.04.1996**
(21) Anmeldenummer: 91918521.5
(22) Anmeldetag: 02.11.1991
(51) Int. Cl.: C07C 227/08, C07C 229/62

(54) **VERFAHREN ZUR HERSTELLUNG VON 2,5-DI-PHENYLAMINO-TEREPHTHALSÄURE UND IHRER DIALKYLESTER IN HOHER REINHEIT**
METHOD OF PREPARING 2,5-DI(PHENYLAMINO)TEREPHTHALIC ACID AND ITS DIALKYL ESTERS AS HIGH-PURITY PRODUCTS
PROCEDE DE PRODUCTION D'ACIDE 2,5-DI-PHENYLAMINO-TEREPHTALIQUE ET DE SES ESTERS DE DIALKYLE TRES PURS

(30) Priorität: 23.11.1990 DE 4037244; 16.01.1991 DE 4101084
(43) Veröffentlichungstag der Anmeldung: 08.09.1993
(73) Patentinhaber: HOECHST AKTIENGESELLSCHAFT, D-65926 Frankfurt am Main (DE)
(72) Erfinder: ARNDT, Otto, D-6238 Hofheim (DE); FUCHS, Hermann, D-6240 Königstein (DE); GILB, Walter, D-6238 Hofheim (DE)
(86) Internationale Anmeldenummer: EP9102067
(87) Internationale Veröffentlichungsnummer: WO9209558

(56) Entgegenhaltungen:
- EP-A- 0 057 873
- EP-A- 0 363 756
- DE-A- 1 915 436
- FR-A- 1 319 519
- GB-A- 891 640

## Beschreibung

Die vorliegende Erfindung betrifft ein ökologisch und ökonomisch verbessertes Verfahren zur Herstellung von 2,5-Di-phenylamino-terephthalsäure und ihrer Di-alkylester der allgemeinen Formel (1) 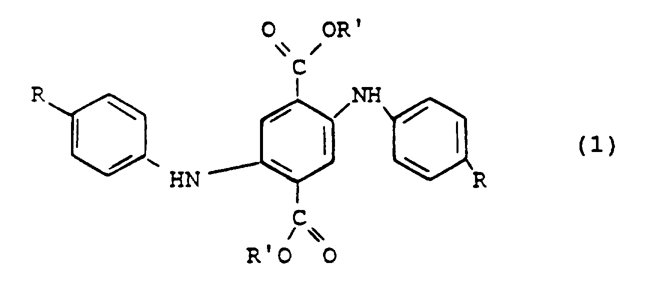 in welcher R ein Wasserstoffatom oder eine Methylgruppe und R' ein Wasserstoffatom oder eine Methyl- oder Ethylgruppe bedeuten.

Es ist bekannt, 2,5-Di-phenylamino-terephthalsäure und deren Di-alkylester nach einem Mehrstufenverfahren herzustellen, indem man nach Art einer Dieckmann- bzw. doppelten Claisen-Kondensation Bernsteinsäure-di-alkylester zum 2,5-Dihydroxy-cyclohexadien-dicarbonsäure-(1,4)-dialkylester cyclisiert (Fortschr. chem. Forschung, Bd. 1 (1950) 685-724), anschließend diesen in den 2,5-Di-phenylamino-dihydro(3,6)-terephthalsäure-di-alkylester durch eine Kondensationsreaktion mit einem primären Phenylamin (beispielsweise Anilin oder Toluidin) in Xylol oder Ethylbenzol oder in Gemischen daraus in Gegenwart einer aliphatischen Säure (beispielsweise Essigsäure) überführt, diesen dehydriert (oxidiert) zum 2,5-Diphenylamino-terephthalsäure-dialkylester, anschließend diesen Ester alkalisch verseift (z.B. in alkoholischer Natronlauge) und durch Behandeln des angefallenen 2,5-di-phenylamino-terephthalsauren Di-Natriumsalzes mit Säure die 2,5-Di-phenylamino-terephthalsäure freisetzt.

Bei der Beschreibung der Herstellung der Di-phenylamino-terephthalsäure nach dem vorstehend beschriebenen Weg aus Bernsteinsäureester werden in der Literatur (JP 49-108 036; DE-OS 1 915 436 und DE-OS 3 834 747 (EP 0 363 756)), eine Reihe von Verfahrensparametern beschrieben, wie beispielsweise Lösemittel; die Zwischenisolierung einzelner oder aller Synthesestufenprodukte (wie (1) Succinoyl-bernsteinsäure-di-alkylester; (2) 2,5-Di-phenylamino-dihydro(3,6)-terephthalsäure-di-alkylester; (3) 2,5-Di-phenylaminoterephthalsäure-di-alkylester; (4) 2,5-Di-phenylamino-terephthalsäure); die Art der angewandten Katalysatoren, gegebenenfalls mit Zusatzstoffen für die vorstehend genannten Zwischenstufen (1), (2) und (3); zeitliche Abfolge von Oxidation und Verseifung (Verseifung gleichzeitig mit Oxidation oder anschließend); Dehydrierungs-(Oxidations-)mittel (wie beispielsweise Nitrobenzol und dessen Derivate, Chinone, Sauerstoff, Jod); Aufarbeitung der eingesetzten Hilfsstoffe (wie beispielsweise Lösemittel, Phenylamin (Anilin, p-Toluidin), Katalysatoren, Zusatzstoffe).

Die in der Literatur genannten Verfahren erfüllen nicht zugleich alle Anforderungen an Wirtschaftlichkeit, geringe Umweltbelastung und Qualität des Endprodukts.

Es erweisen sich diejenigen Verfahren vom ökologischen Standpunkt aus als wenig sinnvoll, die als Oxidationsmittel für die Dehydrierungsstufe statt Luft bzw. Sauerstoff Nitroaromaten (z.B. Nitrobenzol und Derivate wie Nitrobenzol-m-sulfonsäure) verwenden.

Ein Stoff wie beispielsweise Nitrobenzol sollte als "Aufbaubestandteil" in Synthesen verwendet werden, jedoch nicht als Hilfsstoff, da dessen Folgeprodukte, wie beispielsweise Anilin, Azo- und Azoxybenzol, aufwendig entfernt werden müssen und dabei wegen starker Verunreinigungen nicht mehr wieder verwertbar sind. Sie würden die Umwelt belasten (Abwasser, Deponie).

Die Verwendung von Sauerstoff ergibt bei Beachtung gewisser Voraussetzungen nur Wasser als Folgeprodukt.

Auch solche Verfahren, die Luft mit Zusatzstoffen von der Art der Chinone und ihrer Derivate (Sulfonsäuren, Chloranil), quaternäre Ammoniumsalze (DE-AS 1 144 285; EP 57873; EP 363 756) verwenden, sind aus denselben Gründen abzulehnen, da diese Zusatzstoffe im allgemeinen in das Abwasser gelangen und obendrein in einigen Fällen stark wassergefährdend sind. In den Literaturstellen, die Verfahren mit derartigen Zusatzstoffen beschreiben, wird in keinem einzigen Fall deren Rückgewinnung beschrieben.

Die Verwendung nur eines einzigen Lösemittels für alle Synthesestufen ist vorteilhaft.

Die Verwendung von Anilin, Eisessig oder dipolar aprotischen Lösemitteln ist nachteilig, da diese Lösemittel nicht für alle Synthesestufen geeignet sind, beispielsweise
(a) Anilin, Eisessig ungeeignet für Synthesestufe (1),
(b) N-Methylpyrrolidon (NMP) ungeeignet für Synthesestufe (4).

N-Methylpyrrolidon ist zwar in einigen Verfahren für die Synthesestufen (1)-(4) eingesetzt worden. Es ist jedoch bekannt, daß N-Methylpyrrolidon in wäßrig alkalischer Mischung alkaliempfindlich ist (pH > 11,5, NMP-Handbook, GAF Corp. 1972, p. 102). Über die Ausbeute an Recycling-NMP ist in der genannten Literatur keine Aussage gemacht worden (DE-AS 1 082 907, JP 52-005739, JP 60-092245, Org. Prep. Proced. Int. 4 (1) 1972, 1).

Bevorzugt werden bei den in der Literatur beschriebenen Verfahren Aromaten, vorzugsweise Xylol, gelegentlich auch Methanol.

Bei einigen Verfahren wird für die Synthesestufe (1) Xylol mit nennenswerten Mengen einer zweiten Lösemittel-Komponente (z.B. Dimethylsulfoxid, siehe JP 52-059 135) verwendet. Diese zweite Komponente wird im allgemeinen vor Eintritt in die Synthesestufe (2) zusammen mit dem Salzanteil aus der Synthesestufe (1) (z.B. Natriumsulfat) entfernt und gelangt ins Abwasser.

Somit sind nur solche Verfahren empfehlenswert, die Xylol allein verwenden, wie z.B. in JP 49-108 036 und DE-OS 1 915 436 beschrieben.

Gemeinsam ist den beiden Verfahren der beiden vorstehend zitierten Literaturstellen der Einsatz einer isolierten Zwischenstufe und die Verwendung von Luft (nicht Sauerstoff 100 %).

Das Verfahren von JP 49-108 036 geht vom isolierten Produkt von Synthesestufe (2) aus. Das Verfahren von DE-OS 1 915 436 geht von isoliertem Produkt der Synthesestufe (1) aus.

Beide Verfahren beziehen nicht die Synthesestufe (1) ein.

### A.) Zwischenisolierung des Produkts aus Synthesestufe (1):

Grundsätzlich ist ein Verfahren ohne Isolierung der Zwischenstufen wirtschaftlicher als ein Verfahren, bei dem eine oder mehrere Zwischenisolierungen erfolgen. Dies gilt aber nur dann, wenn die Qualität des Endprodukts der Soll-Qualität High-Chem-Produkts entspricht. Bei einem über mehrere Synthesestufen verlaufenden Verfahren ist es sinnvoll, bereits in der ersten Stufe anfallende Nebenkomponenten möglichst frühzeitg, also am besten schon vor Eintritt in die zweite Stufe, zu entfernen.

Stellt man den Succinylobernsteinsäuredimethylester (Produkt der Synthesestufe (1)) nach einem der Verfahren der beschriebenen Literaturstellen in Xylol her und isoliert ihn durch Wasserdampfdestillation des Xylols, so erhält man ihn in guter Qualität. Das Abwasser aus der Wasserdampfdestillation (= "Mutterlauge") ist jedoch biologisch schlecht abbaubar.

Will man diese Abwasserbelastung von biologisch schlecht abbaubaren Komponenten vermeiden und verzichtet auf die "reinigende" Zwischenisolierung, so erhält man oberhalb von 85 °C eine xylolische Lösung des Produkts der Stufe (1) verunreinigt mit diesen Komponenten. Diese Nebenkomponenten bewirken einen deutlichen zusätzlichen Bedarf an Phenylamin, beispielsweise Anilin, p-Toluidin, in der Synthesestufe (2), wodurch auch der Anteil an nicht wasserlöslichen Nebenkomponenten in der Syntehsestufe (3) deutlich erhöht wird. In diesem Fall könnte eine Zwischenisolierung des Produkts aus Synthesestufe (2) sinnvoll sein. Da jedoch auch in der nachfolgenden Synthesestufe (3) durch die Einwirkung von Sauerstoff zusätzlich nennenswerte Nebenkomponenten entstehen, muß auch das Produkt aus der Synthesestufe (3) zwischenisoliert werden.

### B.) Zwischenisolierung des Produkts von Synthesestufe (3) zwecks Reinigung:

Es wurde festgestellt, daß nach der Wasserdampfdestillation des Xylols, Filtration des Di-phenylamino-terephthalsäure-di-alkylesters und Heißwäsche mit Wasser sich alle Verunreinigungen im Nutschgut durch anschließendes Ausblasen des Di-Esters auf der Nutsche mit Wasserdampf und Wäsche mit Methanol überraschenderweise auf einmal entfernen lassen (Beispiele 1a, 15, 16). Bei den mit Methanol auswaschbaren Verunreinigungen handelt es sich hauptsächlich um biologisch schwer abbaubare Komponenten. Sie können durch Verbrennung entsorgt werden. Die wäßrige Mutterlauge hat eine nahezu 100 %ige biologische Abbaubarkeit (= CSB-Eliminierung).

Überraschend ist dabei, daß eine Heißwäsche mit Wasser allein nicht ausreicht, um z.B. einen stark riechenden Anteil an Verunreinigungen aus dem Nutschgut auszuwaschen. Das gelingt erst nach dem Ausblasen mit Dampf. Das Produkt ist danach geruchsfrei.

Die in JP 52-059 135 angegebene Wäsche der xylolischen Reaktionslösung der Stufe (1) mit wäßriger Natriumhydrogencarbonat-Lösung entspricht der Vorstellung, die Verunreinigungen schon in Stufe (1) zu entfernen. Laborversuche dazu zeigten jedoch, daß die Ausbeute in Stufe (3) erheblich niedriger lag und die Gewichtsmenge an Nebenkomponenten, die sich durch Extraktion des Produkts aus Stufe (3) mit Methanol isolieren lassen, nicht abnahm (vgl. Beispiele 12 und 13).

Die Verlagerung der Reinigungsstufe auf das Produkt von Stufe (3) erweist sich dagegen als eine effektive Reinigungsmethode. Das durch den Stand der Technik (JP 52 059 135) gegebene "Vorurteil" einer Reinigung des Reaktionsgemischs von Produkt der Stufe (1) ist somit überholt.

Alle Verfahren, bei denen die Dehydrierung (Oxidation) und Verseifung gleichzeitig erfolgen, bei denen also die Phase des Produkts der Synthesestufe (3) nur kurzfristig, wenn überhaupt, durchlaufen wird und erst das Produkt der Verfahrensstufe (4) isoliert wird, beinhalten die Mitführung von Nebenkomponenten aus den einzelnen Synthesestufen bis in das Endprodukt.

Es ist nun überraschend, daß die wirksamste Reinigungsmöglichkeit in der Wäsche des Produkts der Synthesestufe (3) liegt, also auf der Synthesestufe, auf welcher die Carbonsäuren als Ester vorliegen, zumal in der nachfolgenden Verseifung zu einer wäßrigen Lösung des Di-Natriumsalzes der Synthesestufe (4) die an sich mögliche Klärung über Aktivkohle dann nicht ausreicht, wenn auf die Zwischenisolierung des Produkts der Synthesestufe (1) verzichtet wird. In DE-OS 38 34 747, Beispiel 4, ist eine Klärung der alkalischen, ethanolisch-wäßrigen Reaktionslöung (pH 12,1) des Di-Natriumsalzes beschrieben. Die Klärung dient jedoch nur dazu, um eine sehr geringe Menge Schwebstoff zu entfernen. Die Hauptmenge der Nebenkomponenten verbleibt in der Lösung (z.B. die gesamte Überschußmenge des Phenylamins, z.B. p-Toluidin und die 2,5-Di-p-toluidino-benzoesäure) und kann somit bei der Fällung der freien Säure deren Qualität negativ beeinflussen.

Es war nicht vorhersehbar, daß in einer Serie von möglichen aufeinanderfolgenden Zwischenstufen gerade die Zwischenstufe des Di-phenylamino-terephthalsäure-di-alkylesters die effektvollste Reinigung ermöglicht. Es war auch nicht voraussehbar, daß nach einer uneffektiven Heißwäsche mit Wasser erst das Ausblasen mit Wasserdampf zu einem geruchsfreien Di-phenylamino-terephthalsäure-di-alkylester führte.

Das vorliegend beschriebene Verfahren beinhaltet eine sinnvolle Aufteilung der Nebenkomponenten auf die Entsorgungsmöglichkeiten, wie Verbrennung von Fabrikationsrückständen und biologische Reinigung des Abwassers, die sich überraschenderweise so ergab, daß die biologisch schlecht abbaubaren Komponenten zur Verbrennung kommen können, die biologisch gut abbaubaren aber ins Abwasser gelangen (im Gegensatz zu z.B. EP 363 756).

### C.) Säure in Synthesestufe (2):

Es wurde gefunden, daß sich der Einsatz von organischer Säure bei Synthesestufe (2) (DE-OS 19 15 436) erheblich reduzieren läßt, wenn man die höher siedende, mit Xylol fast gleich siedende Propionsäure verwendet, weil der mit dem azeotropen Austrag des Reaktionswassers verbundene Austrag an organischer Säure dadurch erheblich reduziert wird. Es wurde ferner gefunden, daß die Propionsäure zwar auch noch in Mengen eingesetzt werden muß, die deutlich höher (100 Mol-%) sind als die Menge, die man üblicherweise bei Katalysatoren gewohnt ist, jedoch läßt sich der größte Teil der Propionsäure im Recycling führen (der Verlust durch Bildung von Propionsäure-anilid bzw. -p-toluidid ist gering).

Hexafluorpropansulfonsäure reagiert zwar weder mit Anilin bzw. p-Toluidin zum Sulfonsäureamid noch mit Xylol zum Sulfon und ist bereits in katalytischer Menge (< 100 Mol-%) sehr geeignet für die Synthesestufe (2), ist jedoch im Abwasser biologisch schwer abbaubar (Beispiel 17).

### D.) Verwendung von reinem Sauerstoff in einer geschlossenen Apparatur:

In JP 49-108 036 und DE-OS 19 15 436 wurde für die Synthesestufe (3) (Dehydrierung (Oxidation)) die Kombination Xylol/Luftsauerstoff beschrieben.

Wie auch bei den anderen Verfahren (z.B. Methanol/Luft; DE-OS 38 34 747) bedingt die Verwendung von Luft, daß in einer offenen Apparatur gearbeitet wird, da der Stickstoffanteil der eingeblasenen Luft als Abgasstrom abgeführt werden muß und somit auch die dem jeweiligen Partialdruck entsprechenden Lösemitteldämpfe, die in technisch aufwendiger Weise aus dem Abgasstrom entfernt werden muß (Kühlanlage, Waschturm) und somit auch auf die gesetzlich vorgeschriebenen Werte herabgesetzt werden müssen. Das ist kostenaufwendig und unwirtschaftlich.

Wir haben nun gefunden, daß sich dieser Nachteil beheben läßt, indem man Sauerstoff 100 % in das Reaktionsgefäß eindosiert und zwar bevorzugt in das Reaktionsgemisch unter dessen Oberfläche. Da kein Abgas entsteht, kann die Apparatur geschlossen bleiben. Es wird bei Normaldruck oder schwach erhöhtem Druck bis ca. 3 bar gearbeitet (vgl. Beispiele 9-11, 18).

Es wurde nun ferner gefunden, daß Sauerstoff in dem in Form von Luft vorliegenden Mengenverhältnis zu Stickstoff (21 Vol-% O₂, 78 Vol.-% N₂) und unter den in der Literatur beschriebenen Bedingungen das Lösemittel Xylol in geringem Maß angreift unter Bildung im wesentlichen vom Oxidationsprodukten des Xylols.

Überraschenderweise ist die Gesamtmenge an Oxidationsprodukten des Xylols und die Anzahl dieser Komponenten bei Einsatz von Sauerstoff 100 % im Vergleich zum Einsatz von Luft nicht angestiegen (ca. 0,1 Gew.-% bezogen auf Xylol).

Ein weiterer Vorteil des Einsatzes von Sauerstoff 100 % im Vergleich zu Luft ist die Möglichkeit der Begrenzung des Sauerstoffgehalts in der Atmosphäre über dem Reaktionsgemisch auf einen Wert der unterhalb des Explosionsbereichs des Systems Xylol/N₂/O₂ liegt, d.h. unterhalb ca. 9 Vol.-%.
Der Sauerstoff wird in dem Maße eindosiert, wie es der aktuelle Sauerstoffbedarf erfordert bei Einhaltung einer konstanten Sauerstoff-Konzentration im Gasraum über dem Reaktionsgemisch.

Die Eindosierungsgeschwindigkeit wird über die Messung der Sauerstoffkonzentration im Gasraum über dem Reaktionsgemisch gesteuert (vgl. Beispiel 18).

Ein weiterer Vorteil des Einsatzes von Sauerstoff 100 % im Vergleich zu Luft ist, daß die durch das Eingasrohr (Tauchrohr) eingeblasene Gasmenge nur ein Fünftel derjenigen der Luft ist, entsprechend dem Gehalt von ca. 21 Vol-% Sauerstoff in Luft.
Bei Luft führt die hohe Gasströmung durch das Tauchrohr zur Verdampfung von Lösemittel und damit zur Bildung eines Feststoffpfropfens, der das Tauchrohr an seinem Ende rasch verstopft, selbst bei glockenförmiger Erweiterung des Tauchrohrendes. Wegen der Reduzierung des Gasstromes auf ein Fünftel tritt beim Einblasen von Sauerstoff 100 % keine Verstopfung mehr auf.

Ein weiterer Vorteil des Einsatzes von "Sauerstoff 100 %" im Vergleich zu Luft besteht darin, daß bei gleichem, auf Sauerstoff bezogenen Volumenstrom die Reaktionsdauer der Oxidation des Produkts der Synthesestufe (2) kleiner ist als beim Einblasen von Luft (vgl. Beispiele 9 und 10).

### E.) Anwendung von Katalysatoren:

Es wurde weiterhin gefunden, daß der Umsatz des Produkts der Synthesestufe (2) mit dem Sauerstoff in einer ersten Zeitphase bis zu ca. 50 % Umsatz linear mit der Zeit verläuft (ca. 4-5 Stunden bei Luft bzw. ca. 1-3 Stunden bei O₂ 100 %), solange noch das genannte Produkt in ausreichender Menge als Bodenkörper vorlag. Es folgt dann als 2. Phase eine Unregelmäßigkeit, die sich in einer Verlangsamung des Umsatzes äußert. In der 3. und letzten Phase geht schließlich der Umsatz beschleunigt bis zum Ende der Reaktion (ca. 8-9 Stunden bei Luft bzw. ca. 6-7 Stunden bei O₂ 100 %) weiter.

Besonders in der 2. und 3. Phase muß die Sauerstoffdosierung genau kontrolliert werden, um zu verhindern, daß der O₂-Gehalt im Gasraum über dem Reaktionsgemisch auf über 8 Vol-% ansteigt und somit in den Explosionsbereich des Systems Xylol/N₂/O₂ gelangt. Von Vorteil wäre nun ein zügigerer Verlauf des Umsatzes in der 2. Phase.

In einer großen Anzahl von Laborversuchen im Glasrührkolben zeigte sich ein schwacher positiver Effekt hinsichtlich der Glättung der Umsatzkurven bei Anwesenheit von V4A-Stahl (als Netz um den Rührer gespannt) (Beispiel 3). Dieser Effekt verbesserte sich nach Zugabe eines Gemisches aus Molybdänpulver und Molybän(VI)-oxid, besonders aber auf Zusatz von Samarium(III)-oxid oder Vanadin(IV)-oxidacetylacetonat (Beispiele 1b, 6-8, 10). Durch den zügigeren und gleichmäßigeren Verlauf des Umsatzes ist die Einhaltung einer konstanten O₂-Konzentration im Gasraum über dem Reaktionsgemisch leichter, somit auch die Gefahr, in den Explosionsbereich zu gelangen, geringer. Die Wirkung des Katalysators äußert sich in einer Steigerung der Verfahrenssicherheit.

Beobachtet wurde eine weitere Wirkung der Katalysatoren: Der Anteil an biologisch nicht mehr abbaubaren Stoffen in den Abwässern der Synthesestufe (3) wird überraschenderweise erheblich erniedrigt (Beispiele 1a/2, 9/11).

Die 2,5-Di-phenylamino-terephthalsäure wird nach dem erfindungsgemäßen Verfahren in guten Ausbeuten und hoher Reinheit in einem auf ökologischen Fortschritt bedachten Verfahren erhalten. Sie ist geeignet als Ausgangsprodukt zur Herstellung von Chinacridon-Pigmenten.

Gegenstand der vorliegenden Erfindung ist somit ein verbessertes Verfahren zur Herstellung von 2,5-Di-phenylamino-terephthalsäure und ihrer Di-alkylester der allgemeinen Formel (1) 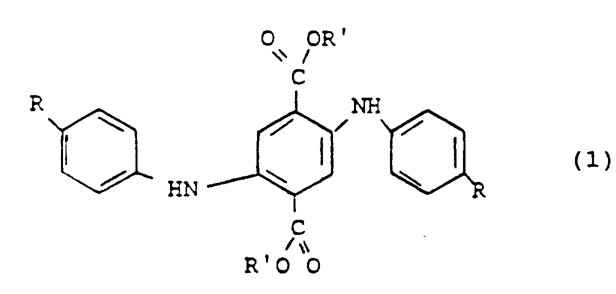 in welcher R ein Wasserstoffatom oder eine Methylgruppe und R' ein Wasserstoffatom oder eine Methyl- oder Ethylgruppe bedeuten, durch (1) Umsetzung von Bernsteinsäure-di-alkyl(C₁-C₂)-ester nach Art einer Dieckmann-Kondensation mit Natrium-alkoholat in Xylol zum Di-Natrium-Salz des 2,5-Dihydroxy-cyclohexadien-dicarbonsäure-(1,4)-dialkyl (C₁-C₂)esters, (2) Umsetzung des so erhaltenen Kondensationsprodukts nach Zersetzen des Di-Natrium-Salzes mit Säure mit einem Phenylamin der allgemeinen Formel (2) 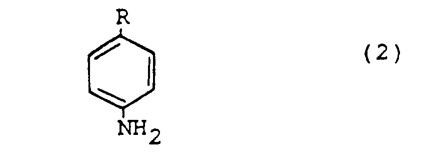 in welcher R die vorstehend genannte Bedeutung hat, in Gegenwart einer organischen Säure in Xylol zum 2,5-Di-phenylamino-dihydro(3,6)-terephthalsäure-di-alkyl (C₁-C₂)ester, (3) Dehydrierung(Oxidation) des so erhaltenen Cyclohexadien-1,4-derivats mit Sauerstoff zum entsprechenden 2,5-Di-phenylamino-terephthalsäure-di-alkyl (C₁-C₂)ester, (4) gegebenenfalls Verseifung des so erhaltenen Dialkylesters in methanolischer Natronlauge zum entsprechenden 2,5-di-phenylamino-terephthalsauren Di-Natrium-Salz und (5) Freisetzung der 2,5-Di-phenylamino-terephthalsäure aus dem genannten Di-Natriumsalz mit Säure, in dem man den in Stufe (1) anfallenden 2,5-Dihydroxy-cyclohexadien-dicarbonsäure-(1,4)-di-alkyl(C₁-C₂)ester nicht aus seinem Reaktionsgemisch in Xylol isoliert, für die Umsetzung mit dem Phenylamin der genannten Formel (2) in Stufe (2) Propionsäure als Säurekatalysator verwendet, die Dehydrierung(Oxidation) des erhaltenen 2,5-Diphenylamino-dihydro-(3,6)-terephthalsäure-di-alkyl(C₁-C₂)esters in Stufe (3) mit 100 %igem Sauerstoff in einer geschlossenen Apparatur in Gegenwart eines Katalysators aus V₄A-Stahl und/oder aus einem Übergangsmetall des Periodischen Systems der Elemente, wie beispielsweise Molybdän oder Vanadin oder
beispielsweise Samarium oder dessen Verbindungen, wie beispielsweise Samarium(III)-oxid, oder Mischungen daraus, unter Konstanthaltung des Sauerstoffgehalts der Gasatmosphäre unterhalb 8 Volumprozent über dem Reaktionsgemisch vornimmt, den erhaltenen 2,5-Di-phenylamino-terephthalsäure-di-alkyl(C₁-C₂)ester durch Filtration aus wäßrigem Medium zwischenisoliert, beispielsweise durch Abfiltrieren auf einer Nutsche, anschließend den zwischenisolierten Di-alkyl(C₁-C₂)-ester durch Ausblasen mit Wasserdampf auf der Nutsche und anschließende Wäsche mit Methanol oder Ethanol, vorzugsweise Methanol, reinigt.

Durch die nachstehenden Beispiele wird das erfindungsgemäße Verfahren näher erläutert, ohne darauf beschränkt zu werden.

### Beispiel 1a

In einer Glasapparatur mit dicht schließendem Rührer, Wasserauskreiser und Rückflußkühler werden 760 Teile einer 15 %igen xylolischen Lösung (Reaktionsgemisch), entsprechend 114 Teilen 2,5-Dihydroxy-cyclohexadiendicarbonsäure-(1,4)-dimethylester ("SucEst") (hergestellt unter Bedingungen, wie beispielsweise in JP 52-059 135 beschrieben, jedoch ohne Zusatz einer zweiten Lösemittelkomponente), mit 173 Teilen eines Gemisches aus 149 Teilen Recycling-Xylol und 24 Teilen Propionsäure und zusätzlich mit 61 Teilen Propionsäure versetzt. Dann läßt man bei 75 °C unter Inertgasatmosphäre (Stickstoff) 135 Teile p-Toluidin als Schmelze hinzulaufen. Durch langsame Steigerung der Temperatur des Reaktionsgemischs auf ca. 103 °C in ca. 3 Stunden wird die Reaktion zum 2,5-Di-p-toluidino-dihydro(3,6)-terephthalsäuredimethylester beendet. Das entstehende Reaktionswasser wird durch Umpump der Stickstoffatmosphäre über einen Kühler auskondensiert (ca. 2 Stunden).
Anschließend werden als Katalysator 0,1-0,8 Teile Molybdän oder Vanadium(IV)-oxidacetylacetonat zugesetzt oder ein V4A-Drahtnetz um den Rührer gewickelt. Dann leitet man einen gegebenenfalls vorgeheizten Luftstrom von ca. 15 Litern pro Stunde durch ein Tauchrohr ca. 8-9 Stunden bei einer Temperatur von 103 °C ein. Der Abgasstrom wird über einen Ausscheider für Reaktionswasser, über einen Kühler und über einen Waschturm, mit z.B. Polyethylenglykoldimethylether, zur Absorption von Xylol-Emissionen abgeleitet. Im Abgasstrom wird die aktuelle Konzentration des Sauerstoffs gemessen. Sie liegt durchschnittlich bei ca. 15 Vol-%. Bei dieser apparativen Anordnung wird der Sauerstoffgehalt der Luft zu ca. 25 % ausgenutzt. Man zieht stündlich Proben für die Flüssigkeitschromatografie (HPLC) und erstellt somit eine in Gewichtsprozent ausgedrückte Umsatzkurve von Edukt und Dedukt. Die 1. Ableitung (Winkel der Tangenten) sollte stetig abnehmen ab ca. 50 % des Umsatzes. Anschließend destilliert man ca. 20 % des Xylols (149 Teile mit 24 Teilen Propionsäure) ab. Die restliche Hauptmenge des Xylols wird dann durch eine Wasserdampfdestillation abdestilliert, wobei schließlich eine wäßrige Suspension des 2,5-Di-p-toluidino-terephthalsäure-dimethylesters erhalten wird. Das wasserdampfdestillierte Xylol wird gereinigt und für die Herstellung des SucEst eingesetzt. Der 2,5-Di-p-toluidino-terephthalsäure-dimethylester wird durch Filtration über eine beheizte Drucknutsche (vorzugsweise ausgestattet mit einem Rührer) isoliert. Das auf der Nutsche befindliche kristalline Rohprodukt (226 Gewichtsteile, Schmelzpunkt 205-207 °C) wird mit Wasserdampf ausgeblasen, bis es geruchsfrei ist. Anschließend wird mit Methanol gewaschen. Man erhält 195 Teile methanolfeuchten 2,5-Di-p-toluidino-terephthalsäure-dimethylester (= 97 % d.Th., bezogen auf SucEst) mit einem Schmelzpunkt von 215-220 °C.

Außerdem fällt ein salzfreies Abwasser mit nahezu 100 %iger biologischer Abbaurate an (= CSB-Eliminierung). Aus dem Methanolfiltrat lassen sich nach alkalischer Verseifung ca. 20-25 Teile Feststoff isolieren, der nur noch wenig Zielprodukt enthält.
Der methanolfeuchte Di-Ester wird in einem Gemisch aus Recycling-Methanol und Wasser mit 150 Teilen Natronlauge 33 % in einem VA-Rührautoklav bei 106 °C zum Di-Natrium-Salz verseift. Die alkalische Produktlösung (pH 12,5) läuft nach dem Entspannen über ein Klärfilter und wird anschließend destillativ von Methanol befreit. Die nahezu methanolfreie Produktlösung läuft in eine Vorlage, in der sie pH-gesteuert mit konz. Salzsäure gleichzeitig vereinigt wird.

Man erhält 177 Teile 2,5-Di-p-toluidino-terephthalsäure mit einem Schmelzpunkt von 310 °C. Der Reingehalt ist > 99 % (HPLC, Titration). Die Ausbeute beträgt 98 % d.Th., bezogen auf den Di-phenylamino-terephthalsäure-di-methylester. Außerdem fällt ein Natriumchlorid enthaltendes Abwasser mit einer hohen biologischen Abbaurate an. Der Rest-CSB (Chemischer Sauerstoffverbrauch nach der biologischen Reinigung) des gesamten Verfahrens ist sehr niedrig.

### Beispiel 1b

Man verfährt, wie in Beispiel 1a beschrieben, jedoch werden statt 15 Liter Luft/h, 3 Liter O₂/h durch das Reaktionsgemisch und 12 Liter N₂/h über das Reaktionsgemisch gegast. Ferner wird Samarium(III)-oxid als Katalysator benutzt.
Die Oxidationszeit beträgt nach 50 % Umsatz nur 1,5 Stunden, nach 100 % Umsatz nur 5 Stunden.
Die erste Ableitung der Umsatzkurve nimmt auch nach 1,5 Stunden weiterhin stetig ab.

### Beispiel 2

Man verfährt, wie in Beispiel 1a beschrieben, jedoch ohne Zusatz von Katalysator.
Man erhält die gleiche Ausbeute an 2,5-Di-p-toluidino-terephthalsäure-dimethylester und die gleiche Menge an durch Methanol extrahierbaren Nebenkomponenten.
Die Dauer zur vollständigen Oxidation ist um ca. 2 Stunden auf ca. 11 Stunden verlängert. Die 1. Ableitung der Umsatzkurve erreicht nach 50 % des Umsatzes ein Minimum, steigt dann gegen Ende wieder deutlich an.
Die Qualität der 2,5-Di-p-toluidino-terephthalsäure ist etwas schlechter:

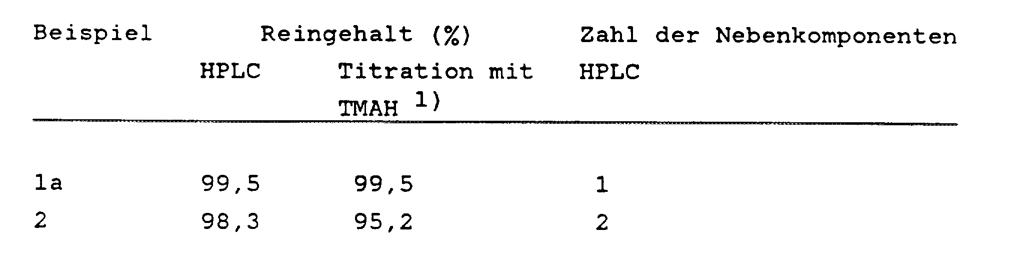

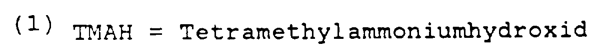

Der biologisch nicht abbaubare organische Anteil (= Rest-CSB) im Abwasser des 2,5-Di-p-toluidino-terephthalsäure-dimethylesters ist deutlich höher:

| Beispiel | Rest-CSB g O₂/kg Ester |
|---|---|
| 1a | 0 |
| 2 | 17 |

### Beispiel 3 (Vergleichsbeispiel)

Man verfährt, wie in Beispiel 2 beschrieben, jedoch bei Abwesenheit von V4A-Stahl-Katalysator während der Synthesestufen (1), (2) und (3). Die Ergebnisse sind wie bei Beispiel 2 mit einer geringfügigen weiteren Verschlechterung des Reingehaltes der Säure (HPLC = 97,4 %) und des Rest-CSB (= 23 g O₂/kg Di-Ester).

### Beispiel 4

Man verfährt, wie in Beispiel 1a beschrieben, jedoch mit Molybdän(V)-chlorid als Katalysator (0,150 Gewichtsteile). Man erhält eine gegenüber Beispiel 1a noch verbesserte Qualität der 2,5-Di-p-toluidino-terephthalsäure:

| Beispiel | Reingehalt (%) | | Zahl der Nebenkomponenten HPLC |
|---|---|---|---|
| | HPLC | Titration mit TMAH | |
| 1a | 99,5 | 99,5 | 1 |
| 4 | 100,0 | 99,6 | 0 |

### Beispiel 5

Man verfährt, wie in Beispiel 1a beschrieben, startet die Synthese jedoch mit 114 Gewichtsteilen von reinem 2,5-Dihydroxy-cyclohexadien-dicarbonsäure-(1,4)-dimethylester und nur 129 Gewichtsteilen p-Toluidin.
Man erhält dieselbe Ausbeute an Ester und Säure wie in Beispiel 1a (195 bzw. 177 Gewichtsteile), jedoch eine erniedrigte Menge an durch Methanol extrahierbaren Nebenkomponenten und eine bessere Qualität der Di-phenylamino-terphthalsäure. Ferner benötigt man eine kürzere Oxidationszeit.

| Bsp. | Oxidationszeit durch Methanol | | | Saüre | |
|---|---|---|---|---|---|
| | (Stunden) nach Umsatz | | extrahierbare Nebenkomponenten (nach alkalischer Verseifung) Gew.Teile | Reingehalt (%) HPLC | Zahl der Nebenkomponenten HPLC |
| | 50 % | 100 % | | | |
| 1a | 5 | 9 | 21,5 | 99,5 | 1 |
| 5 | 2 | 6 | 7,4 | 100,0 | 0 |

### Beispiele 6-8 (Beispiel 8 Vergleichsbeispiel)

Man verfährt, wie in Beipsiel 5 beschrieben, jedoch mit anderen bzw. ohne Katalysatoren bei der Oxidation:

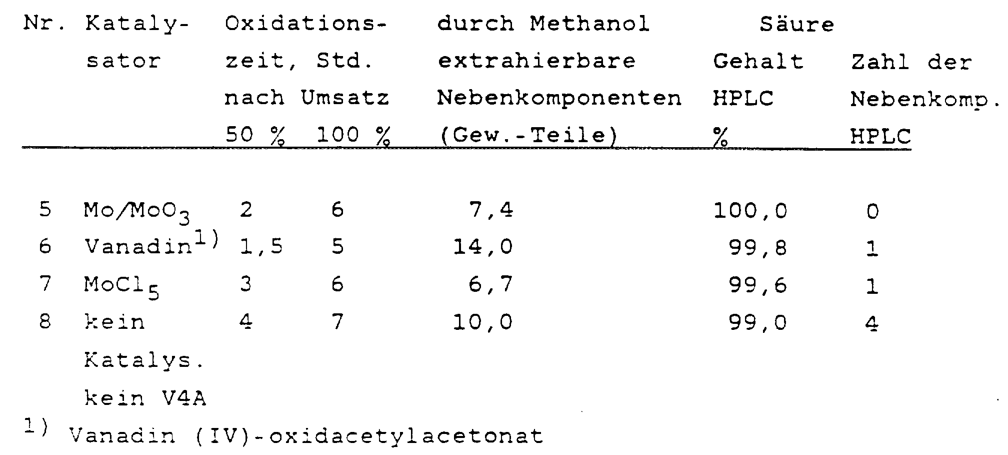

In den Beispielen Nr. 5-7 nimmt die 1. Ableitung der Umsatzkurve weiterhin stetig ab. Beispiel 8 verhält sich wie in Beispiel 2 beschrieben.

### Beispiele 9-11

Man verfährt, wie in Beispiel 5 beschrieben, jedoch werden statt 15 Liter Luft/h 3 Liter O₂/h durch das Reaktionsgemisch und 12 Liter N₂/h über das Reaktionsgemisch geleitet:

| Bsp. | Oxidation mit | Katalysator | Oxidationszeit (Std) | | Rest-CSB g O₂/kg Est |
|---|---|---|---|---|---|
| | | | nach Umsatz von | | |
| | | | 50 % | 100 % | |
| 5 | Luft | Mo/MoO₃ | 2 | 6 | - |
| 9 | Sauerstoff 100 % | Mo/MoO₃ | 1,5 | 5 | 5 |
| 10 | Sauerstoff 100 % | Sm₂O₃ | 1 | 5 | - |
| 11 | Sauerstoff 100 % | kein Katalysator | 2,5 | 7 | 18 |

Man erkennt deutlich die kürzeren Oxidationszeiten bei den Beispielen 9 und 10. Die Ausbeuten an Di-Ester und Säure sind wie bei Beispiel 1a.

### Beispiele 12 und 13

Man verfährt, wie in Beispiel 1a beschrieben, jedoch mit folgenden Abänderungen:
1) Die xylolische Reaktionslösung des 2,5-Dihydroxy-cyclohexadien-(1,3)-dicarbonsäure-(1,4)-dimethylesters (SucEst) wird mit 250 Teilen einer 10%igen wäßrigen, heißen Natriumhydrogencarbonatlösung gewaschen (Beispiel 12) bzw., alternativ, mit 250 Teilen heißen Wassers (Beispiel 13).
2) Die Oxidation wird mit Sauerstoff 100 % gemäß Beispielen 9-11 ausgeführt.

Die Ausbeuten an 2,5-Di-p-toluidino-terephthalsäure und seines Di-methylesters sinken im Falle der Wäsche des SucEst mit Hydrogencarbonat deutlich ab. Die mit Methanol aus dem Ester extrahierbaren Nebenkomponenten sind nicht herabgesetzt worden:

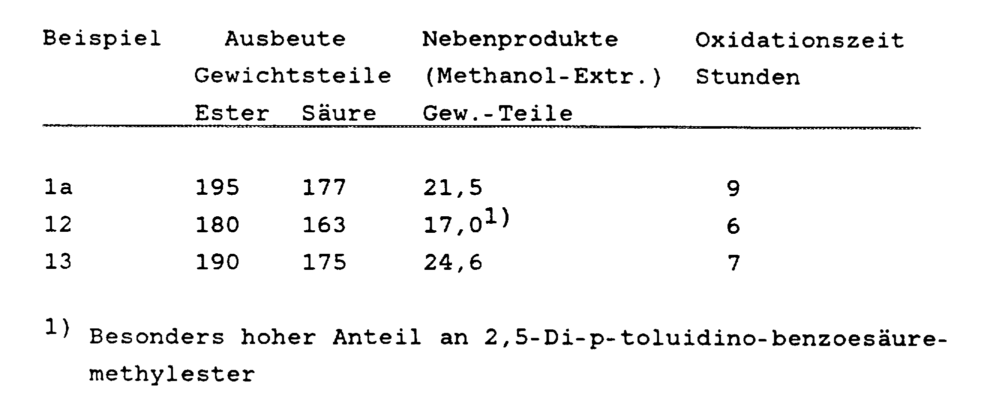

### Beispiel 14

Man verfährt, wie in Beispiel 2 beschrieben, jedoch werden nur 129 Teile p-Toluidin eingesetzt. Man erhält 191 Teile Di-Ester, 175 Teile Säure und 25 Teile Methanol-Extrakt. Statt 5,5 Teilen p-Toluidin wurde im Wasserdampfdestillat des Xylols kein p-Toluidin wiedergefunden.
Die Oxidationszeit ist jedoch etwas kürzer (statt 11 nur 8 Stunden).

Dieser Versuch zeigt, daß 129 Teile p-Toluidin die untere Menge des Einsatzes darstellen (theoretische Einsatzmenge 107 Teile). Die Qualität der 2,5-Di-p-toluidino-terephthalsäure ist wie in Beispiel 2.

### Beispiele 15 und 16

Man verfährt, wie in Beispiel 8 beschrieben, jedoch setzt man in Beispiel 16 für die Verseifung den 2,5-Di-p-toluidino-terephthalsäure-di-methylester in der Form ein, wie man ihn aus der Wasserdampfdestillation des xylolischen Reaktionsgemischs gemäß Beispiel 1a erhält (215 Gew.-Teile, Schmelzpunkt 205-210 °C), d.h. ohne die dort beschriebenen, aufeinanderfolgenden Reinigungsschritte
(a) Ausblasen des Di-Esters auf dem Filterorgan mit Wasserdampf
(b) Extrahieren des Di-Esters mit Methanol.

Die alkalische Verseifungslösung wird heiß über Aktivkohlepulver geklärt.
Man erhält 186 Teile 2,5-Di-p-toluidino-terephthalsäure schlechter Qualität.
Alternativ wurde ein Versuch ausgeführt (Beispiel 15), bei dem der Reinigungsschritt (a) Ausblasen des Esters auf dem Filterorgan mit Wasserdampf ausgeführt wurde.

Man erhält 179 Teile einer Qualität, die zwischen den Qualitäten von Beispiel 8 und Beispiel 16 liegt.

| Nr. | Ausbeute | | Reingehalt (%) | | Nebenkomponenten (HPLC) | |
|---|---|---|---|---|---|---|
| | Rohester | Säure | HPLC | Titration TMAH | Menge (%) | Anzahl |
| | Gewichtsteile | | | | | |
| 8 | 209 | 177,0 | 99,0 | 99,8 | 1,0 | 4 |
| 15 | 206 | 179,0 | 97,5 | 97,0 | 2,5 | 3 |
| 16 | 215 | 186,0 | 91,2 | 93,0 | 8,8 | 6 |

Man erkennt, daß die Hauptmenge der in der 2,5-Di-p-toluidino-terephthalsäure enthaltenen Verunreinigungen durch das Ausblasen des Di-Esters mit Wasserdampf entfernt wird.

### Beispiel 17 (Vergleichsbeispiel)

Man verfährt, wie in Beispiel 5 beschrieben, gibt jedoch statt der 85 Teile Propionsäure 58 Teile Hexafluorpropansulfonsäure ("HFPS") als Hilfsmittel für die Kondensation des SucEst mit p-Toluidin hinzu.
Man erhält trotz vollständiger Kondensation nur 177 Teile 2,5-Di-p-toluidino-terephthalsäure-dimethylester (nach der Methanol-Wäsche), da der Umsatz des Dihydroesters bei der Oxidation selbst nach 14 Stunden noch nicht quantitativ war (es wurden noch 5 % d.Th. des Di-hydroesters laut HPLC gefunden). An der Wandung des Rührkolbens bildeten sich Krusten, die offenbar einen Anteil an Di-hydroester zurückhielten und somit der Oxidation durch Luftsauerstoff entzogen. Selbst bei Erniedrigung des HFPS-Einsatzes auf ca. 20 Teile war die Oxidationsdauer des Di-hydroesters noch immer > 8 Stunden.

### Beispiel 18

Man verfährt, wie in Beispiel 11 beschrieben, jedoch mit 300 facher Ansatzgröße (entsprechend 34200 Teilen SucEst) in einem geschlossenen V4A-Rührkessel.
Nach der Kondensation zum 2,5-Di-p-toluidino-dihydro-(3,6)-terephthalsäure-dimethylester und Auskreisen des Reaktionswassers im Stickstoff-Umpump über Kühler wird Sauerstoffgas 100 % durch ein Tauchrohr eingeblasen (ca. 3000 Teile). Der Sauerstoffgehalt in der Atmosphäre über dem Reaktionsgemisch wird zwischen 2-6 Vol-% konstant gehalten. Die Dosiergeschwindigkeit des eingeblasenen Sauerstoffs wird durch entsprechende Ventilsteuerung dem aktuellen Sauerstoffbedarf angepaßt. Nach maximal 14 Stunden und dem Einblasen von ca. 3000 Teilen Sauerstoff ist die Oxidation beendet.

Ausbeute und Qualität der 2,5-Di-p-toluidino-terephthalsäure (53100 Teile) und seines Di-methylesters (58500 Teile) entsprechen denen des Beispiel 1a.

### Beispiel 19

Durch Variation der Menge von Recycling-Xylol (erhalten durch mehr oder weniger weitgehende Abdestillation eines Teils des Xylols aus dem fertigen Reaktionsgemisch; die restliche Menge an Xylol wird durch Wasserdampfdestillation regeneriert, siehe Beispiel 1a) kann man den Anteil der Recycling-Propionsäure steuern, da sie fast den gleichen Siedepunkt wie Xylol hat.
Damit werden auch die Werte von DOC und CSB (1) in der Mutterlauge des 2,5-Di-p-toluidino-terephthalsäuredimethylesters unter Zurechnung der Werte für die wäßrige Phase bei der Wasserdampfdestillation des Xylols beeinflußt:

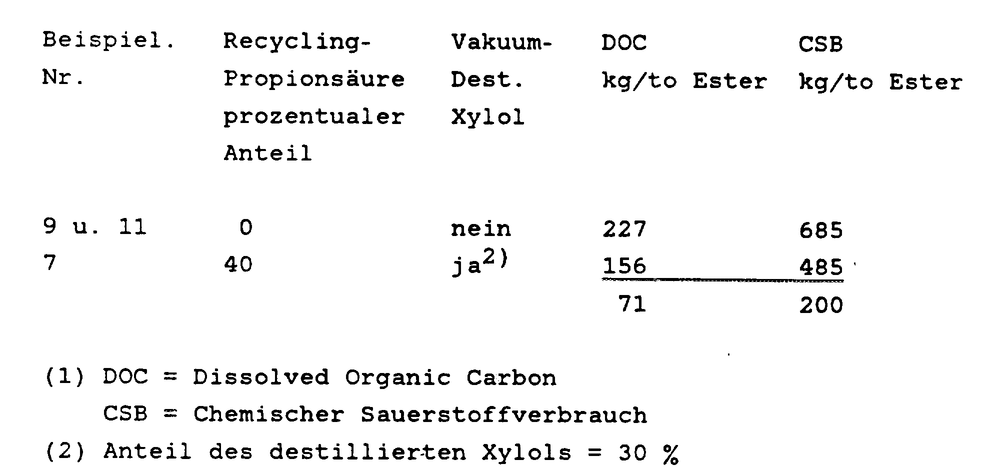

Daraus ist zu entnehmen, daß es aus ökologischen und auch aus ökonomischen Gründen (der CSB verursacht Kosten) vorteilhaft sein kann, nicht das gesamte Xylol durch Wasserdampfdestillation zu regenerieren.

### Beispiel 20

Man verfährt wie in Beispiel 5, setzt jedoch statt p-Toluidin Anilin ein.

Die Einsätze betragen:

| | |
|---|---|
| Succinylobernsteinsaüredimethylester (SucEst) | 114 Teile |
| Anilin | 112 Teile |
| Propionsäure | 90 Teile |
| Xylol | 450 Teile |

Nach Bildung des Produkts aus Synthesestufe 2 (3 Stunden bei 90°C) wird ein Luftstrom von 15 Litern/Stunde bei 97°C 8 Stunden durchgegast.

Man destilliert im Vakuum von ca. 250 mbar bei einer Temperatur bis zu max. 100°C ca. 80 % des Xylols mit einem Gehalt von 73 Teilen Propionsäure (= 80 % des Einsatzes) ab. Die restliche Menge des Xylols wird durch Wasserdampfdestillation zurückgewonnen.
Man isoliert das Produkt (Di-Ester) durch Filtration und wäscht mit heißem Wasser.
Der wasserfeuchte Ester (188 Teile Trockenprodukt, 47 Teile Wasser, Fp = 145-162°C) wird mit 239 Teilen heißem Methanol gewaschen und heiß filtriert.
Man erhält 175 Teile methanolfeuchten 2,5-Di-anilinoterephthalsäuremethylester = 93 % d. Th. bezogen auf SucEst mit einem Schmelzpunkt von 160-164°C. Außerdem fällt ein salzfreies Abwasser mit hoher biologischer Abbaurate (CSB = 100 %) an.
Aus dem Methanolfiltrat lassen sich nach alkalischer Verseifung ca. 4 Teile Feststoff isolieren.
Der methanolfeuchte Ester wird, wie in Beispiel 1a beschrieben, verseift, gefällt und isoliert.
Man erhält 156 Teile 2,5-Di-anilino-terephthalsäure.
Der Reingehalt ist ≥ 99 % (HPLC, Titration).
Die Ausbeute beträgt 96 % d. Th. bezogen auf den Di-Ester bzw. 90 % d. Th. bezogen auf SucEst. Außerdem fällt ein salzhaltiges Abwasser mit einer hohen biologischen Abbaurate an (CSB-Eliminierung = 93 %).

### Beispiel 21

Man verfährt wie in Beispiel 20, jedoch wird der rohe, wasserfeuchte Ester (188 Teile Trockenprodukt) auf der Nutsche nicht mit Methanol gewaschen.
Nach Verseifung erhält man 164 Teile 2,5-Di-anilinoterephthalsäure. Der Reingehalt ist 97 - 98 % (HPLC, Titration).

Qualitätsvergleich gemäß HPLC (Flächen %):

| Beispiel Nr. | Hauptbestandteil | Zahl der Nebenkomponenten |
|---|---|---|
| 20 | 98,6 | 2 |
| 21 | 97,2 | 4 |

Den Einfluß der mit Methanol extrahierbaren Nebenkomponenten auf das Abwasser zeigt folgender Vergleich:

| Beispiel Nr. | CSB kg O₂ | Rest-CSB kg O₂ | DOC kg C pro Tonne Säure |
|---|---|---|---|
| 20 | 206 | 15 | 116 |
| 21 | 250 | 36 | 125 |

Da die mit Methanol extrahierbaren Nebenkomponenten verbrannt werden können, ist durch die Methanol-Wäsche ein deutlicher Beitrag zu Entlastung des Abwassers gegeben.

### Beispiel 22

Man verfährt wie in Beispiel 20, man benutzt jedoch eine Apparatur für automatische Prozeßführung.
Außerdem verwendet man bei der Oxidation (Dehydrierung) 0,54 Teile Vanadin(IV)-oxidacetylacetonat als Katalysator. Die Oxidationsdauer beträgt statt 8 nur noch 6 Stunden. Man erhält 183 Teile wasserfeuchten Ester (Fp = 145-154°C). Der wasserfeuchte Ester wird, wie in Beispiel 1a beschrieben, verseift, gefällt und isoliert.
Man erhält 159 Teile 2,5-Di-anilino-terephthalsäure. Der Reingehalt ist 98 % (Titration).

## Patentansprüche

1. Verfahren zur Herstellung von
2,5-Di-phenylamino-terephthalsäure und ihrer Di-alkylester der allgemeinen Formel (1) 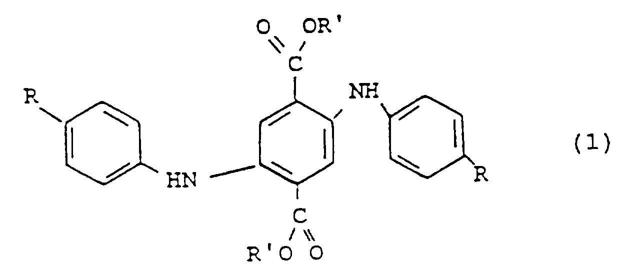 in welcher R ein Wasserstoffatom oder eine Methylgruppe und R' ein Wasserstoffatom oder eine Methyl- oder Ethylgruppe bedeuten, durch (1) Umsetzung von Bernsteinsäure-di-alkyl(C₁-C₂)-ester nach Art einer Dieckmann-Kondensation mit Natrium-alkoholat in Xylol zum Di-Natrium-Salz des
2,5-Dihydroxy-cyclohexadien-dicarbonsäure-(1,4)-dialkyl (C₁-C₂)esters, (2) Umsetzung des so erhaltenen Kondensationsprodukts nach Zersetzen des Di-Natrium-Salzes mit Säure mit einem Phenylamin der allgemeinen Formel (2) 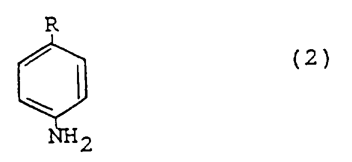 in welcher R die vorstehend genannte Bedeutung hat, in Gegenwart einer organischen Säure in Xylol zum
2,5-Di-phenylamino-dihydro(3,6)-terephthalsäure-di-alkyl (C₁-C₂)-ester, (3) Dehydrierung(Oxidation) des so erhaltenen Cyclohexadien-1,4-derivats mit Sauerstoff zum entsprechenden
2,5-Di-phenylamino-terephthalsäure-di-alkyl (C₁-C₂)-ester, gegebenenfalls (4) Verseifung des so erhaltenen Dialkylesters in methanolischer Natronlauge zum entsprechenden 2,5-di-phenylamino-terephthalsauren Di-Natrium-Salz und
2,5-Di-phenylamino-terephthalsäure aus dem genannten Di-Natriumsalz mit Säure, dadurch gekennzeichnet, daß man den in Stufe (1) anfallenden
2,5-Dihydroxy-cyclohexadien-dicarbonsäure(1,4)-di-alkyl (C₁-C₂)-ester nicht aus seinem Reaktionsgemisch in Xylol isoliert, für die Umsetzung mit dem Phenylamin der genannten Formel (2) in Stufe (2) Propionsäure als Säurekatalysator verwendet, die Dehydrierung(Oxidation) des erhaltenen 2,5-Diphenylamino-dihydro-(3,6)-terephthalsäure-di-alkyl (C₁-C₂)-esters in Stufe (3) mit 100 %igem Sauerstoff in einer geschlossenen Apparatur in Gegenwart eines Katalysators aus V₄A-Stahl und/oder aus einem Übergangsmetall des Periodischen Systems der Elemente oder dessen Verbindungen oder Mischungen daraus, unter Konstanthaltung des Sauerstoffgehalts der Gasatmosphäre unterhalb 8 Volumprozent über dem Reaktionsgemisch vornimmt, den erhaltenen 2,5-Di-phenylamino-terephthalsäure-di-alkyl(C₁-C₂)-ester durch Filtration aus wäßrigem Medium zwischenisoliert, anschließend den zwischenisolierten Di-alkyl(C₁-C₂)-ester durch Ausblasen mit Wasserdampf auf der Nutsche und anschließende Wäsche mit Methanol oder Ethanol reinigt.

2. Verfahren nach Anspruch 1, dadurch gekennzeichnet, daß man die Dehydrierung (Oxidation) des 2,5-Di-phenylaminodihydro-(3,6)-terephthalsäure-di-alkyl(C₁-C₂)-esters in Gegenwart von Molybdän, Vanadin, Samarium, MoO₃, MoCl₅, Vanadin(IV)-oxidacetylacetonat oder Sm₂O₃ oder Mischungen daraus als Katalysatoren durchführt.

## Claims

1. A process for the preparation of 2,5-di-phenylamino-terephthalic acid or one of its di-alkyl esters, of the formula (1) 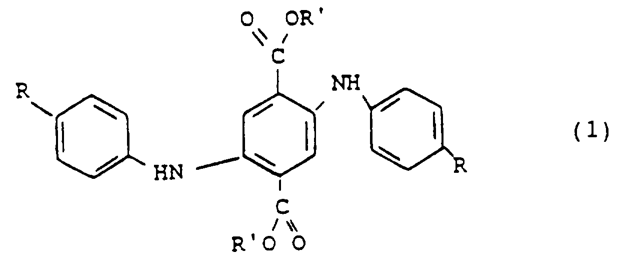 in which R is a hydrogen atom or a methyl group and R' is a hydrogen atom or a methyl or ethyl group, by (1) reaction of a succinic acid di-alkyl(C₁-C₂) ester by a Dieckmann condensation with a sodium alcoholate in xylene to give the di-sodium salt of the 2,5-dihydroxy-cyclohexadiene-1,4-dicarboxylic acid dialkyl(C₁-C₂) ester, (2) reaction of the condensation product thus obtained, after decomposition of the di-sodium salt with an acid, with a phenylamine of the formula (2) 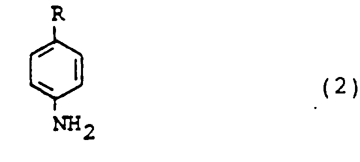 in which R has the abovementioned meaning, in the presence of an organic acid in xylene to give the 2,5-di-phenylamino-3, 6-dihydro-terephthalic acid di-alkyl(C₁-C₂) ester, (3) dehydrogenation (oxidation) of the 1,4-cyclohexadiene derivative thus obtained with oxygen to give the corresponding 2,5-di-phenylamino-terephthalic acid di-alkyl(C₁-C₂) ester, if appropriate (4) hydrolysis of the dialkyl ester thus obtained in methanolic sodium hydroxide solution to give the corresponding 2,5-di-phenylamino-terephthalic acid di-sodium salt and 2,5-di-phenylaminoterepthalic acid from said di-sodium salt with an acid, which comprises not isolating the 2,5-dihydroxy-cyclohexadiene-1,4-dicarboxylic acid dialkyl(C₁-C₂) ester obtained in stage (1) from its reaction mixture in xylene, using propionic acid as the acid catalyst for the reaction with the phenylamine of said formula (2) in stage (2), carrying out the dehydrogenation (oxidation) of the resulting 2,5-diphenylamino-3,6-dihydro-terephthalic acid dialkyl(C₁-C₂) ester in stage (3) with 100 % pure oxygen in a closed apparatus in the presence of a catalyst of V₄A steel and/or of a transition metal of the periodic table of the elements or a compound thereof, or a mixture thereof, the oxygen content of the gas atmosphere above the reaction mixture being kept constant at below 8 percent by volume, intermediately isolating the resulting 2,5-di-phenylamino-terephthalic acid di-alkyl(C₁-C₂) ester from the aqueous medium by filtration, and subsequently purifying the di-alkyl(C₁-C₂) ester intermediately isolated by blowing out with steam on the suction filter and subsequently washing with methanol or ethanol.

2. The process as claimed in claim 1, wherein the dehydrogenation (oxidation) of the 2,5-di-phenylamino-3,6-dihydro-terephthalic acid di-alkyl(C₁-C₂) ester is carried out in the presence of molybdenum, vanadium, samarium, MoO₃, MoCl₅, vanadium(IV) oxideacetylacetonate or Sm₂O₃ or a mixture thereof as the catalyst.

## Revendications

1. Procédé pour la préparation de l'acide 2,5-di-phénylamino-téréphtalique et de ses esters dialkyliques de formule générale (1) : 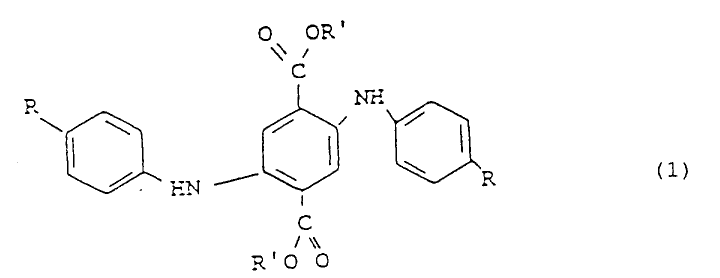 dans laquelle R représente un atome d'hydrogène ou un groupe méthyle et R' représente un atome d'hydrogène ou un groupe méthyle ou éthyle, par (1) réaction de l'ester dialkylique en C₁-C₁₂ de l'acide succinique selon le mode d'une condensation de Dieckmann avec l'alcoolate de sodium dans du xylène pour obtenir le sel disodique de l'ester 1,4-dialkylique en C₁-C₂ de l'acide 2,5-dihydroxy-cyclohexadiène-dicarboxylique, (2) réaction du produit de condensation ainsi obtenu après la décomposition du sel disodique par l'acide avec une phénylamine de formule générale (2) : 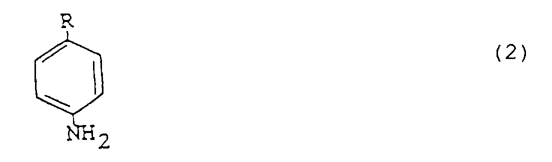 dans laquelle R a la signification donnée ci-dessus, en présence d'un acide organique dans du xylène pour obtenir l'ester dialkylique en C₁-C₂ de l'acide 2,5-di-diphénylaminodihydro-(3,6)-téréphtalique, (3) déshydrogénation (oxydation) du dérivé de cyclohexadiène-1,4 ainsi obtenu par l'oxygène pour obtenir l'ester dialkylique en C₁-C₂ de l'acide 2,5-di-phénylamino-téréphtalique, éventuellement (4) saponification de l'ester dialkylique dans une lessive de soude méthanolée pour obtenir le sel disodique de l'acide 2,5-di-phénylamino-téréphtalique et l'acide 2,5-di-phénylamino-téréphtalique à partir du sel disodique cité à l'aide d'acide, caractérisé en ce que l'ester 1,4-di-alkylique en C₁-C₂ de l'acide 2,5-dihydroxycyclohexadiène dicarboxylique formé à l'étape (1) n'est pas isolé de son mélange réactionnel dans le xylène, on utilise l'acide propionique comme catalyseur acide pour la réaction avec la phénylamine de formule (2) citée dans l'étape (2), on met en oeuvre la déshydrogénation (oxydation) de l'ester dialkylique en C₁-C₂ de l'acide 2,5-di-phénylamino-dihydro-(3,6)-téréphtalique obtenu dans l'étape (3) avec de l'oxygène à 100 %, dans un appareil fermé, en présence d'un catalyseur en acier V4A et/ou d'un métal de transition du Système Périodique des Eléments ou ses dérivés, en maintenant constante, inférieure à 8% en volume, la teneur en oxygène dans l'atmosphère gazeuse au-dessus du mélange réactionnel, en isolant de façon intermédiaire à par filtration partir du milieu aqueux l'ester dialkylique en C₁-C₂ de l'acide 2,5-di-phénylamino-téréphtalique, en purifiant l'ester dialkylique en C₁-C₂ isolé de façon intermédiaire par soufflage avec de la vapeur d'eau sur le filtre en verre fritté et ensuite par lavage avec du méthanol ou de l'éthanol.

2. Procédé selon la revendication 1, caractérisé en ce que l'on met en oeuvre la déshydrogénation (oxydation) de diester alkylique en C₁-C₂ de l'acide 2,5-di-phénylamino-dihydro-(3,6)-téréphtalique en présence de molybdène, de vanadium, de samarium, de MoO₃, de MoCl₅, d'acétylacétonate d'oxyde de vanadium(IV) ou de Sm₂O₃ ou de leurs mélanges en tant que catalyseurs.
